# EUROPEAN PATENT APPLICATION

(11) **EP 4 841 261 A1**
(43) Date of publication of application: **18.11.2026**
(21) Application number: 26177193.5
(22) Date of filing: 07.05.2026
(51) Int. Cl.: G01N 33/543, B01L 3/00, C12Q 1/6816, G01N 1/30, G01N 1/31, G01N 33/558, B01L 7/00, G01N 1/40

(54) **CONTACTLESS ANTIBODY PROCESSING APPARATUS**

(30) Priority: 13.05.2025 US 202563804674 P
(71) Applicant: Cytoaurora Biotechnologies, Inc., Zhubei City, Hsinchu County (TW)
(72) Inventor: HUANG, Chung-Er, Zhubei City (TW); CHEN, Chien-Hung, Zhubei City (TW); HUANG, Yen-Sen, Zhubei City (TW); HUANG, Yun-Yen, Zhubei City (TW); HUANG, Wei-Chiao, Zhubei City (TW)
(74) Representative: Viering, Jentschura & Partner mbB Patent- und Rechtsanwälte

(57) **Abstract**

A contactless antibody processing apparatus (1000) includes a specimen supplying device (200), a capturing device (100), and a power device (400). The specimen supplying device (200) is configured to input a liquid specimen (S) having a plurality of cells (P) and a plurality of antibodies (A) into the capturing device (100). The capturing device (100) includes a field emission structure (1) and a mating structure (2) that is spaced apart from the field emission structure (1). The power device (400) is electrically coupled to an electrode layer of the field emission structure (1) and an electrode layer of the mating structure (2). The power device (400) is configured to drive the capturing device (100) to enable an end of an insulation layer (14) of the field emission structure (1) to be electropositive for attracting the cells (P) being electronegative to be held thereon and further attracting the antibodies (A) to connect to the cells (P).

## Description

### FIELD OF THE INVENTION

The present invention relates to a processing apparatus, and more particularly to a contactless antibody processing apparatus.

### BACKGROUND OF THE INVENTION

Conventional cell processing devices have been using the same conventional means of connecting antibodies to cells, and are provided without any mechanisms for promoting the binding of antibodies to cells.

### SUMMARY OF THE INVENTION

In response to the above-referenced technical inadequacies, the present invention provides a contactless antibody processing apparatus for effectively improving on the issues associated with conventional cell processing devices.

In order to solve the above-mentioned problems, one of the technical aspects adopted by the present invention is to provide a contactless antibody processing apparatus, which includes a specimen supplying device, a capturing device, a power device, and a staining device. The specimen supplying device is configured to provide a liquid specimen having a plurality of cells and a plurality of antibodies that are unconnected to the plurality of cells. Each of the plurality of cells has a negative charge, and each of the plurality of antibodies has a sense oligonucleotide. The capturing device is configured to accommodate the liquid specimen from the specimen supplying device. The capturing device includes a field emission structure and a mating structure. The field emission structure includes a first substrate, a first electrode layer formed on the first substrate, and a plurality of pillars. The plurality of pillars are arranged on an inner side of the field emission structure and are spaced apart from each other. The mating structure is spaced apart from the field emission structure along a thickness direction. The mating structure includes a second substrate and a second electrode layer that is formed on the second substrate. The power device is electrically coupled to the first electrode layer and the second electrode layer. The power device is configured to drive the capturing device to make a free end of each of the plurality of pillars to have a positive charge, so as to attract and hold the plurality of cells and attract the plurality of antibodies to connect to the plurality of cells. The staining device is in spatial communication with the capturing device. When the plurality of cells are held on the plurality of pillars and are connected with the plurality of antibodies, the staining device is configured to input a plurality of first antisense oligonucleotides, so that the sense oligonucleotide of at least one of the plurality of antibodies connected to the cells is connected to a corresponding one of the plurality of first antisense oligonucleotides for achieving a first staining operation.

In order to solve the above-mentioned problems, another one of the technical aspects adopted by the present invention is to provide a contactless antibody processing apparatus, which includes a specimen supplying device, a capturing device, and a power device. The specimen supplying device is configured to provide a liquid specimen having a plurality of cells and a plurality of antibodies that are unconnected to the plurality of cells. Each of the plurality of cells has a negative charge. The capturing device is configured to accommodate the liquid specimen from the specimen supplying device, and the capturing device includes a field emission structure and a mating structure. The field emission structure includes a first substrate, a first electrode layer formed on the first substrate, and an insulation layer. The insulation layer is arranged on an inner side of the field emission structure. The mating structure is spaced apart from the field emission structure along a thickness direction. The mating structure includes a second substrate and a second electrode layer that is formed on the second substrate. The power device is electrically coupled to the first electrode layer and the second electrode layer. The power device is configured to drive the capturing device to make an end of the insulation layer have a positive charge, so as to attract and hold the plurality of cells and attract the plurality of antibodies to connect to the plurality of cells.

In order to solve the above-mentioned problems, yet another one of the technical aspects adopted by the present invention is to provide a contactless antibody processing apparatus, which includes a specimen supplying device, a capturing device, and a power device. The specimen supplying device is configured to provide a liquid specimen having a plurality of antibodies. Each of the plurality of antibodies has a sense oligonucleotide having a negative charge. The capturing device is configured to accommodate the liquid specimen from the specimen supplying device, and the capturing device includes a field emission structure and a mating structure. The field emission structure includes a first substrate, a first electrode layer formed on the first substrate, and a plurality of pillars. The plurality of pillars are arranged on an inner side of the field emission structure and are spaced apart from each other. The mating structure is spaced apart from the field emission structure along a thickness direction. The mating structure includes a second substrate and a second electrode layer that is formed on the second substrate. The power device is electrically coupled to the first electrode layer and the second electrode layer. The capturing device is configured to allow a tissue to be disposed therein and located on the second substrate, and the power device is configured to drive the capturing device to make a free end of each of the plurality of pillars have a negative charge, so as to repel and drive the plurality of antibodies to move and connect to the tissue.

Therefore, the contactless antibody processing apparatus provided by the present invention is capable of utilizing the structural configuration of the capturing device to enable the end of the insulation layer (e.g., the free ends of the pillars) in an electropositive mode to effectively achieve the holding of the cells and the connection thereof with the antibodies, or to enable the end of the insulation layer (e.g., the free ends of the pillars) in an electronegative mode to effectively achieve the connection of the tissue and the antibodies.

Furthermore, through the first staining operation performed on each of the cells, the contactless antibody processing apparatus provided by the present invention can obtain the staining images corresponding to biological characterization expressions of each of the cells or the tissue, which facilitates subsequent various assessments and determinations.

These and other aspects of the present invention will become apparent from the following description of the embodiment taken in conjunction with the following drawings and their captions, although variations and modifications therein can be effected without departing from the scope of the novel concepts of the invention.

### BRIEF DESCRIPTION OF THE DRAWINGS

The described embodiments can be better understood by reference to the following description and the accompanying drawings, in which:
FIG. 1 is a schematic perspective view of a contactless antibody processing apparatus according to a first embodiment of the present invention;
FIG. 2 is a schematic cross-sectional view taken along line II-II of FIG. 1;
FIG. 3 is a schematic cross-sectional view showing a subsequent operation of the contactless antibody processing apparatus shown in FIG. 2;
FIG. 4 is a schematic cross-sectional view showing a subsequent operation of the contactless antibody processing apparatus shown in FIG. 3;
FIG. 5 is a schematic cross-sectional view showing a subsequent operation of the contactless antibody processing apparatus shown in FIG. 4;
FIG. 6 is a schematic cross-sectional view showing a subsequent operation of the contactless antibody processing apparatus shown in FIG. 5;
FIG. 7 is a schematic cross-sectional view showing a subsequent operation of the contactless antibody processing apparatus shown in FIG. 6;
FIG. 8 is a schematic cross-sectional view showing a subsequent operation of the contactless antibody processing apparatus shown in FIG. 7;
FIG. 9 is a schematic cross-sectional view of the contactless antibody processing apparatus according to a second embodiment of the present invention;
FIG. 10 is a schematic cross-sectional view showing a subsequent operation of the contactless antibody processing apparatus shown in FIG. 9;
FIG. 11 is a schematic cross-sectional view showing a subsequent operation of the contactless antibody processing apparatus shown in FIG. 10;
FIG. 12 is a schematic cross-sectional view showing a subsequent operation of the contactless antibody processing apparatus shown in FIG. 11; and
FIG. 13 is a schematic cross-sectional view showing a subsequent operation of the contactless antibody processing apparatus shown in FIG. 12.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

### [First Embodiment]

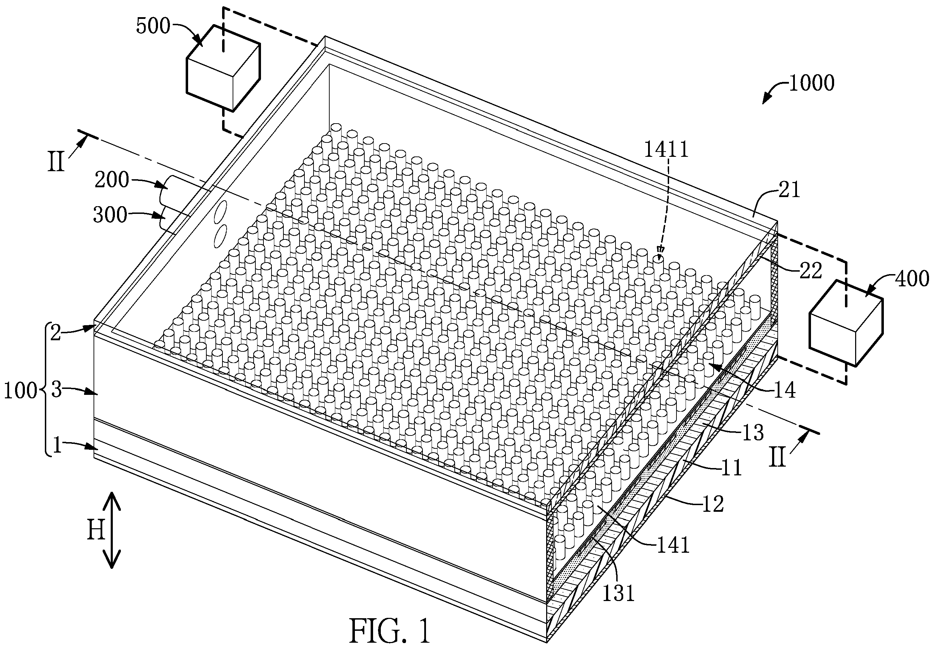

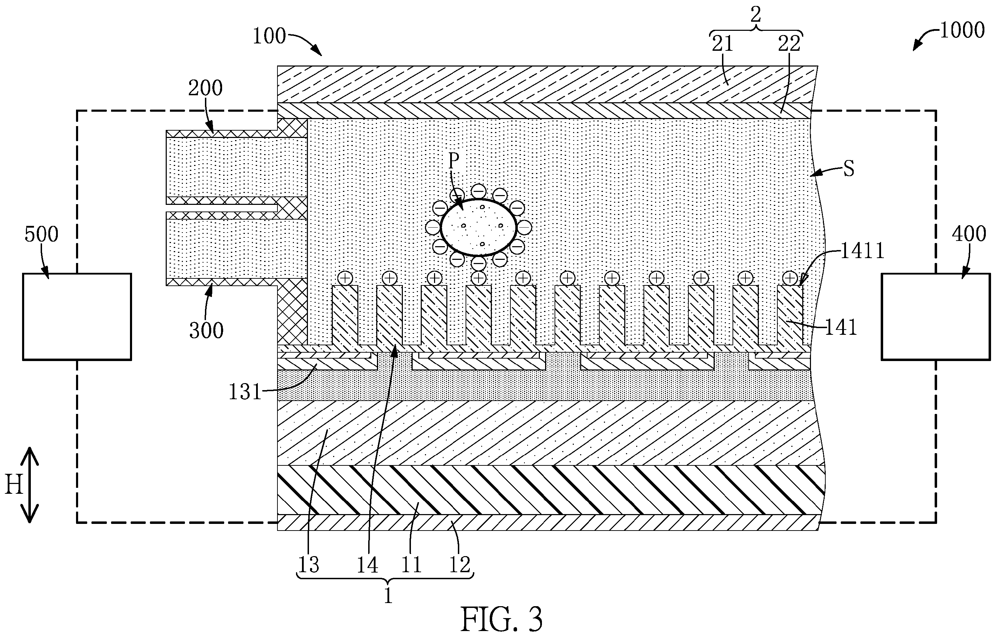

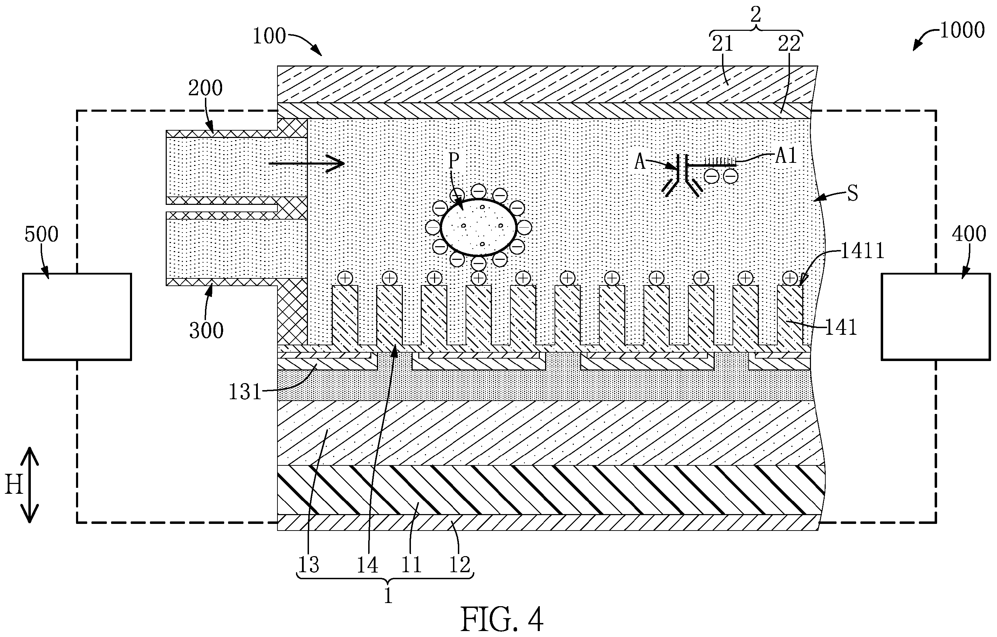

Reference is made to FIG. 1 to FIG. 8, in which a first embodiment of the present invention is provided. As shown in FIG. 1, the present embodiment provides a contactless antibody processing apparatus 1000, which includes a capturing device 100, a specimen supplying device 200 being in spatial communication with the capturing device 100, a staining device 300 being in spatial communication with the capturing device 100, a power device 400 (e.g., an alternating-current device) electrically coupled to the capturing device 100, and a temperature control device 500 that is electrically coupled to the capturing device 100, but the present invention is not limited thereto. For example, in other embodiments of the present invention not shown in the drawings, the staining device 300 and/or the temperature control device 500 can be omitted or replaced by other mechanisms according to practical requirements.

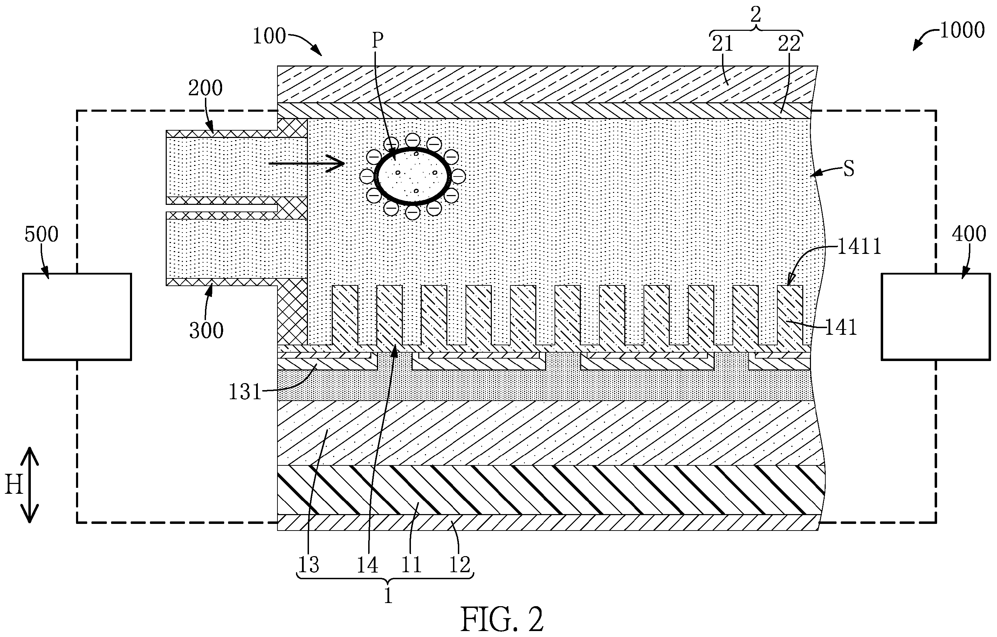

As shown in FIG. 2 to FIG. 5, the specimen supplying device 200 is configured to provide a liquid specimen S having a plurality of cells P and a plurality of antibodies A that are unconnected to the cells P. Each of the cells P has a negative charge, each of the antibodies A has a sense oligonucleotide A1, and the sense oligonucleotide A1 can have a negative charge. However, for the purpose of facilitating the description and understanding of the present embodiment, the drawings only show a portion of the structure of the contactless antibody processing apparatus 1000 and a corresponding portion of the liquid specimen S that has one of the cells P and one of antibodies A.

It should be noted that, the specimen supplying device 200 in the present embodiment inputs the liquid specimen S having the cells P but not yet containing the antibodies A, and then inputs the liquid specimen S having the antibodies A at an appropriate time. Alternatively, in other embodiments of the present invention not shown in the drawings, the specimen supplying device 200 can input the liquid specimen S having the cells P and the antibodies A according to practical requirements.

The liquid specimen S can be a bodily fluid specimen derived from an animal (e.g., blood, lymph, saliva, or urine), and the cells P can be specific types of cells or cell clusters, such as circulating tumor cells (CTCs), fetal nucleated red blood cells (FNRBCs), or bacteria, but the present invention is not limited thereto. For example, in other embodiments of the present invention not shown in the drawings, the liquid specimen S can be a liquid specimen derived from a plant.

The capturing device 100 is a rectangular structure of chip-scale size and is configured to accommodate the liquid specimen S from the specimen supplying device 200. The capturing device 100 includes a field emission structure 1, a mating structure 2 spaced apart from the field emission structure 1 along a thickness direction H, and a bonding layer 3 (e.g., as shown in FIG. 1) that is assembled to a periphery of the field emission structure 1 and a periphery of the mating structure 2. The field emission structure 1 is located below the mating structure 2, at least one of the field emission structure 1 and the mating structure 2 is transparent, and the field emission structure 1 and the mating structure 2 in the present embodiment are two plate-shaped structures that are parallel to each other and that are spaced apart from each other by a distance being greater than the size of any one of the cells P, but the present invention is not limited thereto.

The field emission structure 1 includes a first substrate 11, a first electrode layer 12 formed on the first substrate 11, a semiconductor layer 13 formed on the first substrate 11, and an insulation layer 14 that is formed on the semiconductor layer 13, but the present invention is not limited thereto. For example, in other embodiments of the present invention not shown in the drawings, the semiconductor layer 13 of the field emission structure 1 can be omitted or replaced by other structures according to practical requirements.

In the present embodiment, the first electrode layer 12 is formed on a bottom side of the first substrate 11, and the semiconductor layer 13 is formed on a top side of the first substrate 11 (e.g., the first electrode layer 12 and the semiconductor layer 13 are respectively formed on two opposite sides of the first substrate 11). The insulation layer 14 formed on the semiconductor layer 13 is arranged on an inner side of the field emission structure 1. Specifically, the insulation layer 14 includes a plurality of pillars 141 (e.g., the pillars 141 arranged adjacent to the semiconductor layer 13) that are arranged on the inner side of the field emission structure 1 and that are spaced apart from each other, and the semiconductor layer 13 has a plurality of transistors 131 in a matrix arrangement. The semiconductor layer 13 can adopt an NPN transistor configuration, a PNP transistor configuration, an NP diode configuration, or a PN diode configuration according to practical requirements, but the present invention is not limited thereto. In other words, according to practical requirements, the semiconductor layer 13 (e.g., the transistors 131) provided by the present embodiment can be driven to provide a corona discharge function for the cells P.

The mating structure 2 includes a second substrate 21 and a second electrode layer 22 that is formed on the second substrate 21, and the second electrode layer 22 faces the field emission structure 1 (e.g., the insulation layer 14). In the present embodiment, the power device 400 is electrically coupled to the first electrode layer 12 of the field emission structure 1 and the second electrode layer 22 of the mating structure 2. Accordingly, the power device 400 is configured to drive the capturing device 100 to make an end of the insulation layer 14 (e.g., free ends 1411 of the pillars 141) have a positive charge, so as to attract and hold the cells P and attract the antibodies A to connect to the cells P.

Specifically, the specimen supplying device 200 in the present embodiment first inputs the liquid specimen S having the cells P but not yet containing the antibodies A. After the free end 1411 of each of the pillars 141 has a positive charge to attract and hold the cells P, the specimen supplying device 200 then inputs the liquid specimen S having the antibodies A, so as to attract the antibodies A to connect to the cells P through the positively charged free ends 1411 of the pillars 141, but the present invention is not limited thereto.

Based on the above description, the contactless antibody processing apparatus 1000 in the present embodiment is capable of utilizing the structural configuration of the capturing device 100 to enable the end of the insulation layer 14 (e.g., the free ends 1411 of the pillars 141) in an electropositive mode to effectively achieve the holding of the cells P and the connection thereof with the antibodies A.

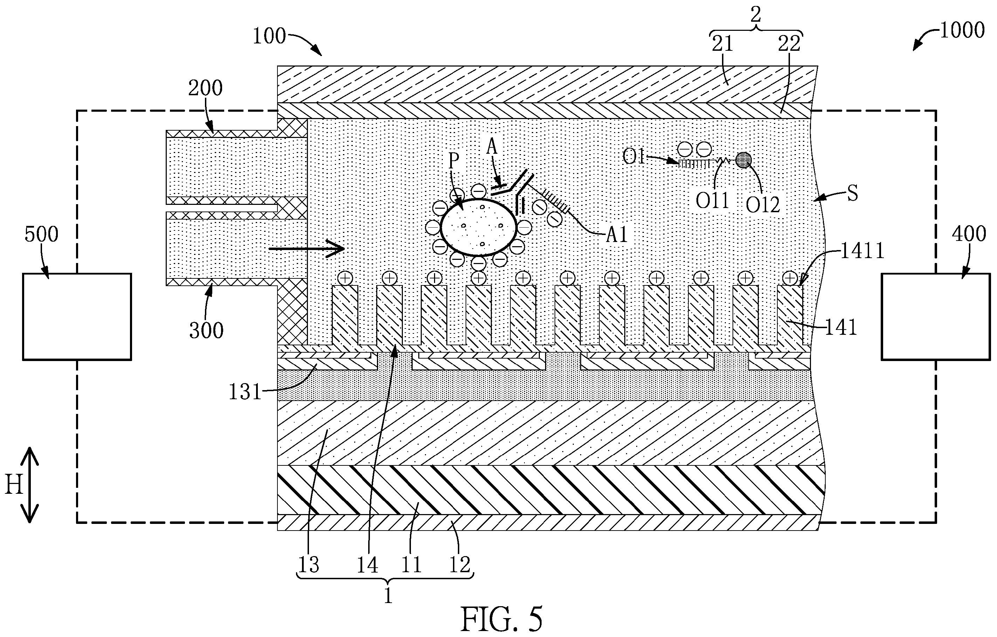

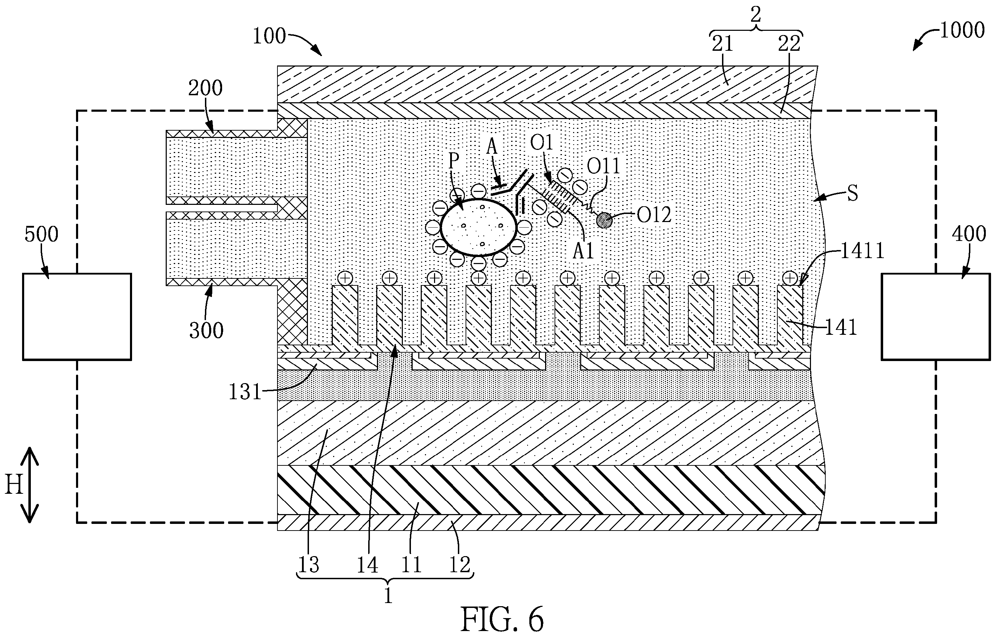

As shown in FIG. 5 and FIG. 6, when the cells P are held on the pillars 141 (e.g., the free ends 1411) and are connected with the antibodies A, the staining device 300 is configured to input a plurality of first antisense oligonucleotides O1, so that the sense oligonucleotide A1 of at least one of the antibodies A connected to each of the cells P is connected to a corresponding one of the first antisense oligonucleotides O1, thereby achieving a first staining operation.

Accordingly, through the first staining operation performed on each of the cells P, the contactless antibody processing apparatus 1000 can obtain staining images corresponding to biological characterization expressions of each of the cells P, which facilitates subsequent various assessments and determinations. Specifically, each of the first antisense oligonucleotides O1 is connected to a first staining dye O12 through a linker 011, so as to achieve the first staining operation through a color of the first staining dye O12, but the present invention is not limited thereto. For example, in other embodiments of the present invention not shown in the drawings, the first staining dye O12 can be selected according to practical requirements (e.g., the first staining dye O12 can be a magnetic bead or a colored plastic particle).

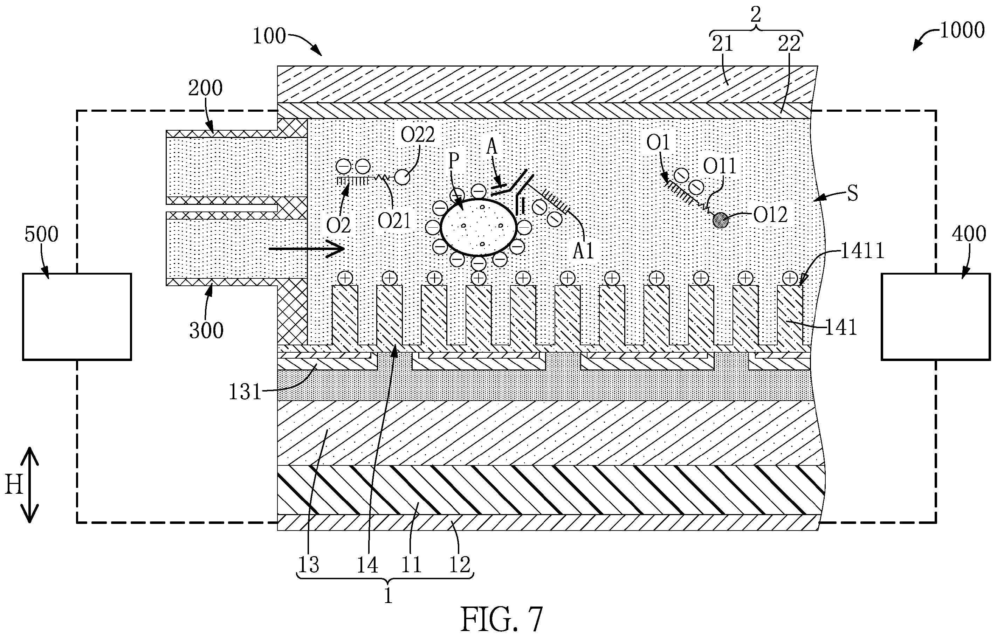

It should be noted that, as shown in FIG. 6, the temperature control device 500 is configured to maintain the capturing device 100 at a first preset temperature that is suitable for the sense oligonucleotide A1 and the corresponding first antisense oligonucleotide O1 to be connected to each other. Furthermore, as shown in FIG. 7, when the first staining operation is completed, the temperature control device 500 is configured to adjust the capturing device 100 to a second preset temperature that is suitable for the sense oligonucleotide A1 and the corresponding first antisense oligonucleotide O1 to be separated from each other, the second preset temperature being higher than the first preset temperature. In the present embodiment, the first preset temperature is substantially greater than 35°C (e.g., the first preset temperature being within a range from 34°C to 39°C), and the second preset temperature is substantially greater than 40°C (e.g., the second preset temperature being within a range from 39°C to 44°C), but the present invention is not limited thereto.

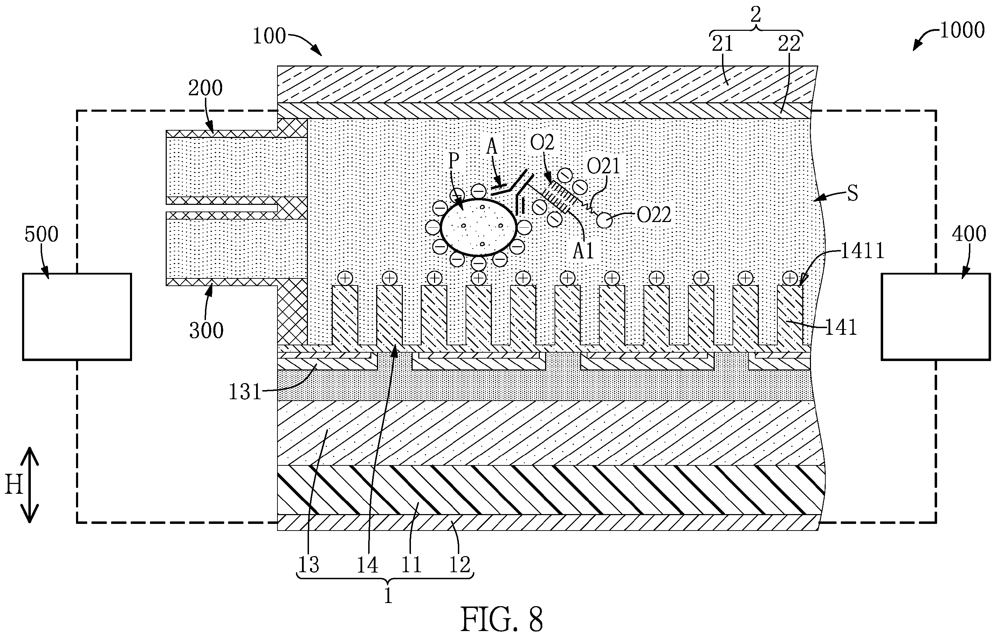

In addition, as shown in FIG. 7 and FIG. 8, when the first staining operation is completed and the sense oligonucleotide A1 and the corresponding first antisense oligonucleotide O1 are separated from each other, the staining device 300 is configured to input a plurality of second antisense oligonucleotides O2, so that the sense oligonucleotide A1 of at least one of the antibodies A connected to each of the cells P is connected to a corresponding one of the second antisense oligonucleotides O2, thereby achieving a second staining operation. Each of the second antisense oligonucleotides O2 is connected to a second staining dye O22 through a linker O21, and the color of the first staining dye O12 is different from a color of the second staining dye O22. The second staining dye O22 can be selected according to practical requirements (e.g., the second staining dye O22 can be a magnetic bead or a colored plastic particle).

Accordingly, the cells P held in the capturing device 100 are capable of undergoing a N number of staining operations through the above-described process, so as to obtain a plurality of staining images respectively corresponding to N types of biological characterization expressions. In the present embodiment, N is a positive integer within a range from 2 to 50, and N is preferably within a range from 4 to 28, but the present invention is not limited thereto. In other words, each staining operation performed on the cells P can correspond to one type of biological characterization expression, which facilitates subsequent various assessments and determinations of the cells P. In addition, the contactless antibody processing apparatus 1000 is further capable of superimposing the staining images to obtain the biological characteristics of the cells P.

### [Second Embodiment]

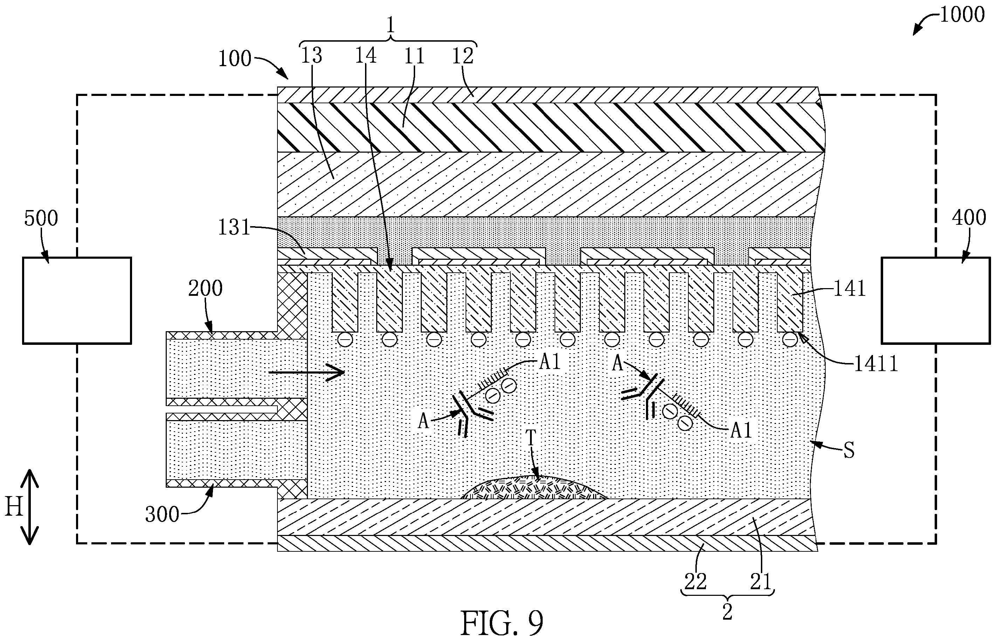

Referring to FIG. 9 to FIG. 13, a second embodiment of the present invention, which is similar to the first embodiment of the present invention, is provided. For the sake of brevity, descriptions of the same components in the first and second embodiments of the present invention will be omitted herein, and the following description only discloses different features between the first and second embodiments.

In the present embodiment, the contactless antibody processing apparatus 1000 includes a capturing device 100, a specimen supplying device 200 being in spatial communication with the capturing device 100, a staining device 300 being in spatial communication with the capturing device 100, a power device 400 (e.g., an alternating-current device) electrically coupled to the capturing device 100, and a temperature control device 500 that is electrically coupled to the capturing device 100, but the present invention is not limited thereto. For example, in other embodiments of the present invention not shown in the drawings, the staining device 300 and/or the temperature control device 500 can be omitted or replaced by other mechanisms according to practical requirements.

It should be noted that the structures and connection relationships of the capturing device 100, the specimen supplying device 200, the staining device 300, the power device 400, and the temperature control device 500 provided by the present embodiment are substantially identical to those described in the first embodiment and will not be described herein.

As shown in FIG. 9, the main difference between the specimen supplying device 200 of the present embodiment and that of the first embodiment lies in that: the liquid specimen S provided by the specimen supplying device 200 in the present embodiment does not include a plurality of cells. Specifically, the specimen supplying device 200 in the present embodiment is configured to provide a liquid specimen S having a plurality of antibodies A, and each of the antibodies A has a sense oligonucleotide A1 with a negative charge.

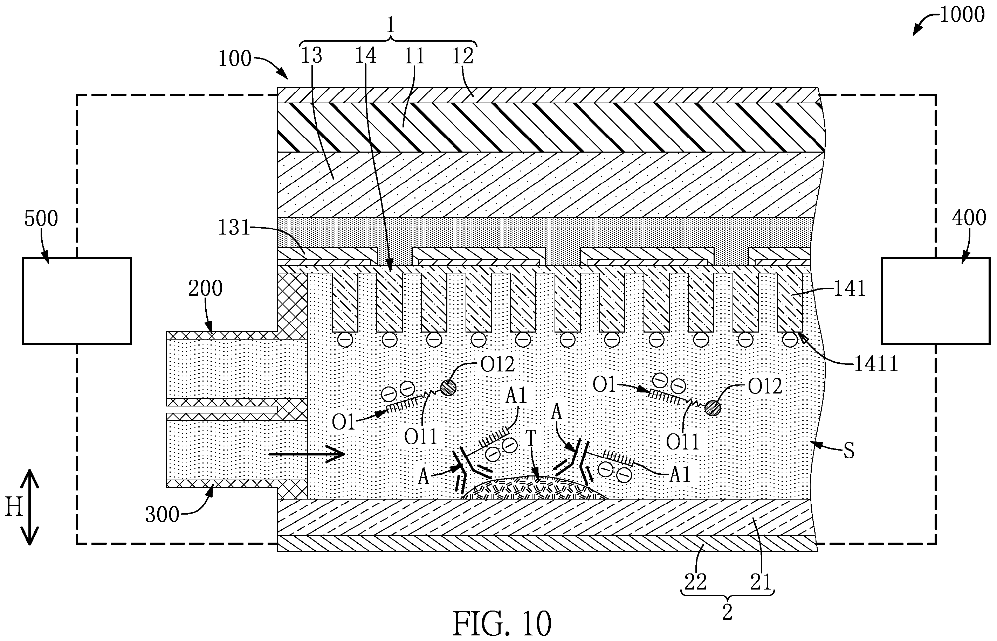

As shown in FIG. 9 and FIG. 10, the main difference between the capturing device 100 of the present embodiment and that of the first embodiment lies in that: the field emission structure 1 is located above the mating structure 2, and the second substrate 21 (e.g., a glass carrier) of the mating structure 2 is arranged on the inner side and faces the insulation layer 14. Accordingly, the capturing device 100 is configured to allow a tissue T to be disposed therein and located on the second substrate 21, and the power device 400 is configured to drive the capturing device 100 to make the end of the insulation layer 14 (e.g., the free ends 1411 of the pillars 141) have a negative charge, so as to repel and drive the antibodies A to move and connect to the tissue T (e.g., cells of the tissue T). The tissue T can be derived from an animal or a plant according to practical requirements, and the present invention is not limited thereto.

Based on the above description, the contactless antibody processing apparatus 1000 in the present embodiment is capable of utilizing the structural configuration of the capturing device 100 to enable the end of the insulation layer 14 (e.g., the free ends 1411 of the pillars 141) in an electronegative mode to effectively achieve the connection of the tissue T and the antibodies A.

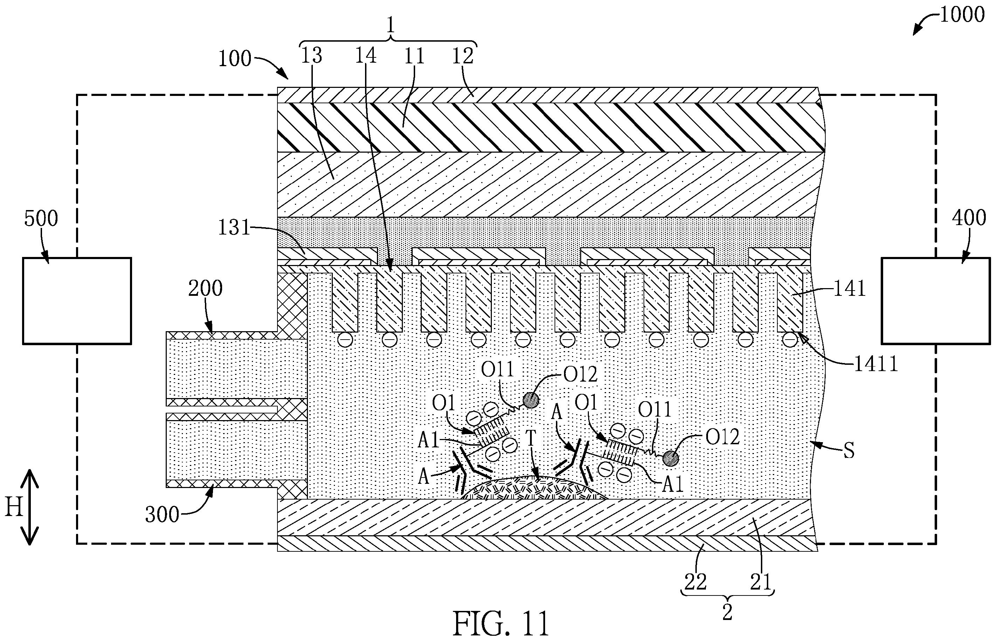

As shown in FIG. 10 and FIG. 11, when the tissue T is disposed on the second substrate 21 and connected with the antibodies A, the staining device 300 is configured to input a plurality of first antisense oligonucleotides O1, so that the sense oligonucleotide A1 of at least one of the antibodies A connected to the tissue T can be connected to a corresponding one of the first antisense oligonucleotides O1, thereby effectively achieving a first staining operation.

Accordingly, through the first staining operation performed on the tissue T, the contactless antibody processing apparatus 1000 in the present embodiment can obtain staining images corresponding to biological characterization expressions of the tissue T, which facilitates subsequent various assessments and determinations. Specifically, each of the first antisense oligonucleotides O1 is connected to a first staining dye O12 through a linker O11, so as to achieve the first staining operation through a color of the first staining dye 012, but the present invention is not limited thereto. For example, in other embodiments of the present invention not shown in the drawings, the first staining dye O12 can be selected in other manners according to practical requirements (e.g., the first staining dye O12 can be replaced by colored plastic particles).

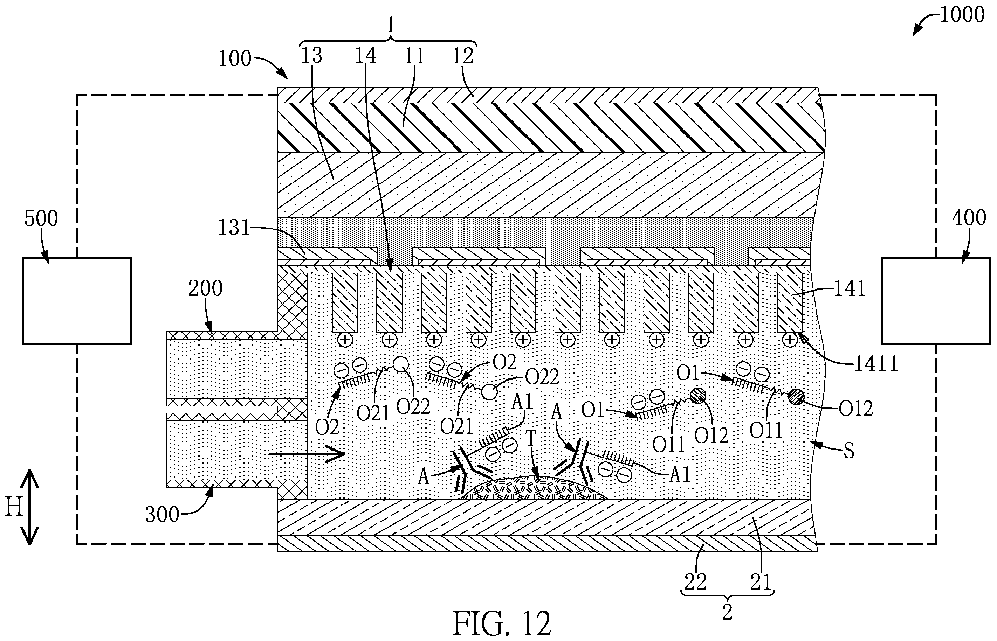

It should be noted that, as shown in FIG. 11, the temperature control device 500 is configured to maintain the capturing device 100 at a first preset temperature that is suitable for the sense oligonucleotide A1 and the corresponding first antisense oligonucleotide O1 to be connected to each other. Furthermore, as shown in FIG. 12, when the first staining operation is completed, the temperature control device 500 is configured to adjust the capturing device 100 to a second preset temperature that is suitable for the sense oligonucleotide A1 and the corresponding first antisense oligonucleotide O1 to be separated from each other, the second preset temperature being higher than the first preset temperature. In the present embodiment, the first preset temperature is substantially greater than 35°C (e.g., the first preset temperature being within a range from 34°C to 39°C), and the second preset temperature is substantially greater than 40°C (e.g., the second preset temperature being within a range from 39°C to 44°C), but the present invention is not limited thereto.

In addition, as shown in FIG. 12, when the first staining operation is completed, the power device 400 is configured to drive the capturing device 100 to make the free ends 1411 of the pillars 141 have a positive charge, so as to attract and separate the corresponding first antisense oligonucleotides O1 from the sense oligonucleotide A1 of at least one of the antibodies A connected to the tissue T.

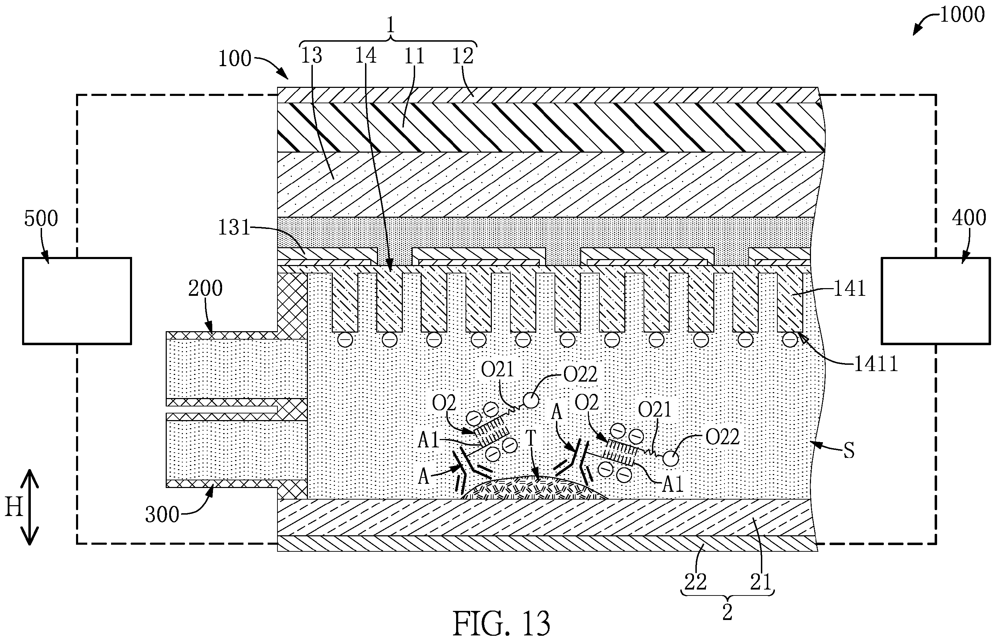

Furthermore, as shown in FIG. 12 and FIG. 13, when the first staining operation is completed and the sense oligonucleotide A1 and the corresponding first antisense oligonucleotide O1 are separated from each other, the staining device 300 is configured to input a plurality of second antisense oligonucleotides O2, so that the sense oligonucleotide A1 of at least one of the antibodies A connected to the tissue T is connected to a corresponding one of the second antisense oligonucleotides O2, thereby achieving a second staining operation. Each of the second antisense oligonucleotides O2 is connected to a second staining dye O22 through a linker O21, and the color of the first staining dye O12 is different from a color of the second staining dye O22.

Accordingly, the tissue T within the capturing device 100 is capable of undergoing a N number of staining operations through the above-described process, so as to obtain a plurality of staining images respectively corresponding to N types of biological characterization expressions. In the present embodiment, N is a positive integer within a range from 2 to 50, and N is preferably within a range from 4 to 28, but the present invention is not limited thereto. In other words, each staining operation performed on the tissue T can correspond to one type of biological characterization expression, which facilitates subsequent various assessments and determinations of the tissue T. In addition, the contactless antibody processing apparatus 1000 is further capable of superimposing the staining images to obtain the biological characteristics of the tissue T.

### [Beneficial Effects of the Embodiments]

In conclusion, the contactless antibody processing apparatus provided by the present invention is capable of utilizing the structural configuration of the capturing device to enable the end of the insulation layer (e.g., the free ends of the pillars) in an electropositive mode to effectively achieve the holding of the cells and the connection thereof with the antibodies, or to enable the end of the insulation layer (e.g., the free ends of the pillars) in an electronegative mode to effectively achieve the connection of the tissue and the antibodies.

Furthermore, through the first staining operation performed on each of the cells, the contactless antibody processing apparatus provided by the present invention can obtain the staining images corresponding to biological characterization expressions of each of the cells or the tissue, which facilitates subsequent various assessments and determinations.

Moreover, through the N number of staining operations, the contactless antibody processing apparatus provided by the present invention can obtain a plurality of staining images respectively corresponding to N types of biological characterization expressions. In other words, each staining operation performed on each of the cells or the tissue can correspond to one type of biological characterization expression, which facilitates subsequent various assessments and determinations of the tissue.

The foregoing description of the exemplary embodiments of the invention has been presented only for the purposes of illustration and description and is not intended to be exhaustive or to limit the invention to the precise forms disclosed. Many modifications and variations are possible in light of the above teaching.

## Claims

1. A contactless antibody processing apparatus (1000), **characterized by** comprising:
a specimen supplying device (200) configured to provide a liquid specimen (S) having a plurality of cells (P) and a plurality of antibodies (A) that are unconnected to the plurality of cells (P), wherein each of the plurality of cells (P) has a negative charge, and each of the plurality of antibodies (A) has a sense oligonucleotide (A1);
a capturing device (100) configured to accommodate the liquid specimen (S) from the specimen supplying device (200), the capturing device (100) including:
a field emission structure (1) including a first substrate (11), a first electrode layer (12) formed on the first substrate (11), and a plurality of pillars (141), wherein the plurality of pillars (141) are arranged on an inner side of the field emission structure (1) and are spaced apart from each other; and
a mating structure (2) spaced apart from the field emission structure (1) along a thickness direction (H), wherein the mating structure (2) includes a second substrate (21) and a second electrode layer (22) that is formed on the second substrate (21);
a power device (400) electrically coupled to the first electrode layer (12) and the second electrode layer (22), wherein the power device (400) is configured to drive the capturing device (100) to make a free end (1411) of each of the plurality of pillars (141) have a positive charge, so as to attract and hold the plurality of cells (P) and attract the plurality of antibodies (A) to connect to the plurality of cells (P); and
a staining device (300) being in spatial communication with the capturing device (100);
wherein, when the plurality of cells (P) are held on the plurality of pillars (141) and are connected with the plurality of antibodies (A), the staining device (300) is configured to input a plurality of first antisense oligonucleotides (O1), so that the sense oligonucleotide (A1) of at least one of the plurality of antibodies (A) connected to the cells (P) is connected to a corresponding one of the plurality of first antisense oligonucleotides (O1) for implementing a first staining operation.

2. The contactless antibody processing apparatus (1000) according to claim 1, further comprising a temperature control device (500) electrically coupled to the capturing device (100), wherein the temperature control device (500) is configured to maintain the capturing device (100) at a first preset temperature that is suitable for the sense oligonucleotide (A1) and the corresponding first antisense oligonucleotide (O1) to be connected to each other.

3. The contactless antibody processing apparatus (1000) according to claim 2, wherein, when the first staining operation is completed, the temperature control device (500) is configured to adjust the capturing device (100) to a second preset temperature that is suitable for the sense oligonucleotide (A1) and the corresponding first antisense oligonucleotide (O1) to be separated from each other, the second preset temperature being higher than the first preset temperature.

4. The contactless antibody processing apparatus (1000) according to claim 3, wherein the first preset temperature is within a range from 34°C to 39°C, and the second preset temperature is within a range from 39°C to 44°C.

5. The contactless antibody processing apparatus (1000) according to claim 3, wherein, when the first staining operation is completed and the sense oligonucleotide (A1) and the corresponding first antisense oligonucleotide (O1) are separated from each other, the staining device (300) is configured to input a plurality of second antisense oligonucleotides (O2) into the capturing device (100), so as to make the sense oligonucleotide (A1) of at least one of the antibodies (A) connected to the cells (P) connected to a corresponding one of the plurality of second antisense oligonucleotides (O2) for achieving a second staining operation.

6. The contactless antibody processing apparatus (1000) according to claim 5, wherein each of the plurality of first antisense oligonucleotides (O1) is connected to a first staining dye (012) through a linker (O11), each of the plurality of second antisense oligonucleotides (O2) is connected to a second staining dye (O22) through a linker (O21), and a color of the first staining dye (O12) is different from a color of the second staining dye (O22).

7. The contactless antibody processing apparatus (1000) according to claim 1, wherein the field emission structure (1) includes a semiconductor layer (13) arranged adjacent to the plurality of pillars (141), and the first electrode layer (12) and the semiconductor layer (13) are respectively formed on two opposite sides of the first substrate (11).

8. A contactless antibody processing apparatus (1000), **characterized by** comprising:
a specimen supplying device (200) configured to provide a liquid specimen (S) having a plurality of cells (P) and a plurality of antibodies (A) that are unconnected to the plurality of cells (P), wherein each of the plurality of cells (P) has a negative charge;
a capturing device (100) configured to accommodate the liquid specimen (S) from the specimen supplying device (200), the capturing device (100) including:
a field emission structure (1) including a first substrate (11), a first electrode layer (12) formed on the first substrate (11), and an insulation layer (14), wherein the insulation layer (14) is arranged on an inner side of the field emission structure (1); and
a mating structure (2) spaced apart from the field emission structure (1) along a thickness direction (H), wherein the mating structure (2) includes a second substrate (21) and a second electrode layer (22) that is formed on the second substrate (21); and
a power device (400) electrically coupled to the first electrode layer (12) and the second electrode layer (22);
wherein the power device (400) is configured to drive the capturing device (100) to make an end of the insulation layer (14) have a positive charge, so as to attract and hold the plurality of cells (P) and attract the plurality of antibodies (A) to connect to the plurality of cells (P).

9. The contactless antibody processing apparatus (1000) according to claim 8, wherein the insulation layer (14) includes a plurality of pillars (141) spaced apart from each other, and the power device (400) is configured to drive the capturing device (100) to make a free end (1411) of each of the plurality of pillars (141) of the insulation layer (14) have the positive charge.

10. The contactless antibody processing apparatus (1000) according to claim 9, wherein the field emission structure (1) includes a semiconductor layer (13) arranged adjacent to the plurality of pillars (141), and the first electrode layer (12) and the semiconductor layer (13) are respectively formed on two opposite sides of the first substrate (11).

11. A contactless antibody processing apparatus (1000), **characterized by** comprising:
a specimen supplying device (200) configured to provide a liquid specimen (S) having a plurality of antibodies (A), wherein each of the plurality of antibodies (A) has a sense oligonucleotide (A1) having a negative charge;
a capturing device (100) configured to accommodate the liquid specimen (S) from the specimen supplying device (200), the capturing device (100) including:
a field emission structure (1) including a first substrate (11), a first electrode layer (12) formed on the first substrate (11), and a plurality of pillars (141), wherein the plurality of pillars (141) are arranged on an inner side of the field emission structure (1) and are spaced apart from each other; and
a mating structure (2) spaced apart from the field emission structure (1) along a thickness direction (H), wherein the mating structure (2) includes a second substrate (21) and a second electrode layer (22) that is formed on the second substrate (21); and
a power device (400) electrically coupled to the first electrode layer (12) and the second electrode layer (22);
wherein the capturing device (100) is configured to allow a tissue (T) to be disposed therein and located on the second substrate (21), and the power device (400) is configured to drive the capturing device (100) to make a free end (1411) of each of the plurality of pillars (141) have a negative charge, so as to repel and drive the plurality of antibodies (A) to move and connect to the tissue (T).

12. The contactless antibody processing apparatus (1000) according to claim 11, wherein each of the plurality of antibodies (A) has a sense oligonucleotide (A1), wherein the contactless antibody processing apparatus (1000) further includes a staining device (300) being in spatial communication with the capturing device (100), and wherein, when the tissue (T) is disposed on the second substrate (21) and connected with the plurality of antibodies (A), the staining device (300) is configured to input a plurality of first antisense oligonucleotides (O1) into the capturing device (100), so that the sense oligonucleotide (A1) of at least one of the antibodies (A) connected to the tissue (T) is connected to a corresponding one of the plurality of first antisense oligonucleotides (O1) for achieving a first staining operation.

13. The contactless antibody processing apparatus (1000) according to claim 12, wherein, when the first staining operation is completed, the power device (400) is configured to drive the capturing device (100) to make the free end (1411) of each of the plurality of pillars (141) have a positive charge, so as to attract and separate the corresponding first antisense oligonucleotides (O1) from the sense oligonucleotide (A1) of at least one of the plurality of antibodies (A) connected to the tissue (T).

14. The contactless antibody processing apparatus (1000) according to claim 13, wherein, when the first staining operation is completed and the sense oligonucleotide (A1) and the corresponding first antisense oligonucleotides (O1) are separated from each other, the staining device (300) is configured to input a plurality of second antisense oligonucleotides (O2) into the capturing device (100), so that the sense oligonucleotide (A1) of at least one of the antibodies (A) connected to the tissue (T) is connected to a corresponding one of the plurality of second antisense oligonucleotides (O2) for implementing a second staining operation.

15. The contactless antibody processing apparatus (1000) according to claim 14, wherein each of the plurality of first antisense oligonucleotides (O1) is connected to a first staining dye (012) through a linker (O11), each of the plurality of second antisense oligonucleotides (O2) is connected to a second staining dye (O22) through a linker (021), and a color of the first staining dye (012) is different from a color of the second staining dye (O22).
